# EUROPEAN PATENT SPECIFICATION

(11) **EP 2 586 474 B1**
(45) Date of publication and mention of the grant of the patent: **24.07.2019**
(21) Application number: 11798113.4
(22) Date of filing: 21.06.2011
(51) Int. Cl.: A61M 1/14, A61M 1/34, A61M 1/36, A61M 1/16

(54) **HEMODIALYSIS DEVICE**
HÄMODIALYSATOR
DISPOSITIF D'HÉMODIALYSE

(30) Priority: 25.06.2010 JP 2010144579
(43) Date of publication of application: 01.05.2013
(73) Proprietor: Nipro Corporation, Osaka-shi, Osaka 531-8510 (JP); Shibuya Kogyo Co., Ltd., Kanazawa-shi, Ishikawa 920-8681 (JP)
(72) Inventor: UEDA, Mitsutaka, Osaka-shi Osaka 531-8510 (JP); SAWADA, Toshiharu, Kanazawa-shi Ishikawa 920-8681 (JP); MITA, Ryuichiro, Kanazawa-shi Ishikawa 920-8681 (JP)
(74) Representative: Mewburn Ellis LLP
(86) International application number: PCT/JP2011/064107
(87) International publication number: WO 2011/162229

(56) References cited:
- JP-A- 10 234 849
- JP-A- 2007 090 058
- JP-A- 2007 105 159
- JP-B2- 4 091 873
- US-A1- 2009 095 679

## Description

### Technical Field

The present invention relates to a hemodialysis apparatus, and in particular, to a hemodialysis apparatus which uses a fresh dialysate that is supplied through a replenishing liquid passage, when the blood is retransfused in a blood circuit which is formed of an artery side passage and a vein side passage, into a patient.

### Background Art

A hemodialysis apparatus is conventionally known which includes: a dialyzer that performs hemodialysis; a dialysate circuit that includes a dialysate supply passage for supplying the fresh dialysate to the dialyzer therethrough and a dialysate recovery passage for recovering a used dialysate which has passed the dialyzer therethrough; and a blood circuit that includes an artery side passage for sending the blood to the dialyzer from the patient therethrough and a vein side passage for returning the blood to the patient from the dialyzer therethrough.

In such a hemodialysis apparatus, a retransfusion that returns a blood remained in the blood circuit to a patient is performed after dialysis treatment, and a hemodialysis apparatus is known which has a bag that stores a normal saline solution connected with the artery side passage, and retransfuses the blood in the blood circuit into the patient by the normal saline solution in this bag at the time of the retransfusion (Patent Literature 1).

On the other hand, there is known another hemodialysis apparatus that performs retransfusion with a different method from that in Patent Literature 1. The method includes: a replenishing liquid passage that connects the dialysate supply passage with the blood circuit to communicate with each other; a replenishing liquid pump which is arranged in the replenishing liquid passage and sends the dialysate from the dialysate supply passage to the blood circuit; and a dialysate filter which is provided in the dialysate supply passage and purifies the dialysate (Patent Literature 2).

The hemodialysis apparatus according to this Patent Literature 2 is structured so as to operate the replenishing liquid pump, when the blood in the blood circuit is retransfused to the patient, pass a fresh dialysate which has been purified by the dialysate filter from the replenishing liquid passage to the artery side passage, and retransfuse the blood in the blood circuit into the patient by the dialysate.

### Prior Art Documents

### Patent Literature

Patent Literature 1: Japanese Patent Laid-Open No. 2007-90058
Patent Literature 2: Japanese Patent No. 4091873

### Summary of Invention

### Problems to be Solved by the Invention

The above described retransfusion operation is performed not only when the dialysis treatment is finished but also when there is no other choice but to stop the treatment as in the power failure.

When a normal saline solution can be supplied from a bag as in the above described apparatus of Patent Literature 1, the apparatus does not particularly cause a problem, but when a dialysate is used for the retransfusion as in the apparatus of Patent Literature 2, the apparatus causes such a problem that a fresh dialysate cannot be supplied due to the power failure, and accordingly the apparatus cannot send a necessary amount of the dialysate to a blood circuit and cannot retransfuse the blood into the patient.

The present invention is designed with respect to such a problem, and provides a hemodialysis apparatus which can promptly retransfuse the blood into the patient even when the power failure has occurred.

### Means for Solving the Problems

Specifically, a hemodialysis apparatus according to claim 1 includes a dialyzer which performs hemodialysis, a dialysate circuit which includes a dialysate supply passage for supplying a fresh dialysate to the dialyzer therethrough and a dialysate recovery passage for recovering a used dialysate therethrough which has passed the dialyzer, a blood circuit which includes an artery side passage for sending blood to the dialyzer from a patient therethrough and a vein side passage for returning the blood to the patient from the dialyzer therethrough, a replenishing liquid passage which connects the dialysate supply passage with the blood circuit to communicate with each other, a replenishing liquid pump which is arranged in the replenishing liquid passage and sends the dialysate from the dialysate supply passage to the blood circuit, a dialysate filter which is provided in the dialysate supply passage and purifies the dialysate, a blood pump which is arranged in the artery side passage, and control means which operates the replenishing liquid pump and the blood pump; and
when the blood in the blood circuit is retransfused into the patient, the control means operate the replenishing liquid pump to pass a fresh dialysate in the dialysate supply passage, which has been purified by the dialysate filter, to the blood circuit through the replenishing liquid passage to retransfuses the blood into the patient, wherein
the hemodialysis apparatus further includes opening means which opens the sealed dialysate circuit to the outside, a storage container which is positioned in a closer side to the dialyzer than the opening means and stores therein the fresh dialysate passing through the dialysate supply passage, and an auxiliary power source for operating at least the replenishing liquid pump, the blood pump, the opening means and the control means; and
an auxiliary power source retransfusing mode is set in the control means as to operate the replenishing liquid pump, the blood pump and the opening means by the auxiliary power source, send the fresh dialysate stored in the storage container to the blood circuit through the replenishing liquid passage, and retransfuse the blood in the blood circuit into the patient.

### Advantageous Effects of Invention

The hemodialysis apparatus according to the present invention sets the auxiliary power source retransfusing mode in the control means and also enables the auxiliary power source to operate the replenishing liquid pump; thereby operates the replenishing liquid pump by the auxiliary power source in the power failure and passes the fresh dialysate stored in the storage container to the blood circuit; and accordingly stops the treatment and can promptly retransfuse the blood into the patient.

### Brief Description of Drawings

Figure 1 is an outside drawing of a hemodialysis apparatus according to the present example.
Figure 2 is a liquid circuit diagram of the hemodialysis apparatus.
Figure 3 is a liquid circuit diagram of a hemodialysis apparatus according to a second embodiment.
Figure 4 is a liquid circuit diagram of a hemodialysis apparatus according to a third example.

### Mode for Carrying out the Invention

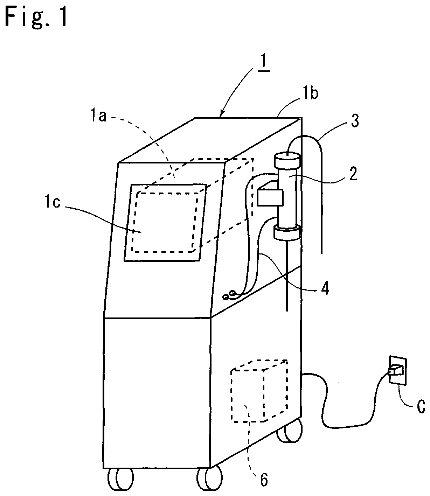

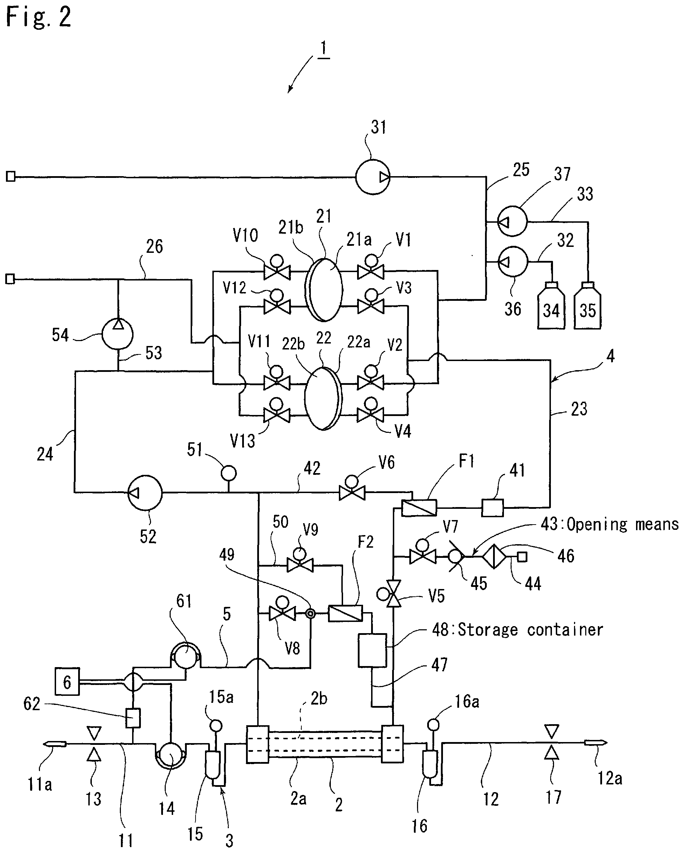

Illustrated embodiments will be described below. Figure 1 illustrates the appearance of a hemodialysis apparatus 1 which performs a dialysis treatment, and Figure 2 illustrates a liquid circuit provided in the inner part of the hemodialysis apparatus 1. This hemodialysis apparatus 1 is structured so as to operate by an electric power supplied from a normal power source such as an outlet C of a hospital, and is controlled by control means 1a which is provided in the apparatus.

The hemodialysis apparatus 1 includes: a dialyzer 2 which is held in the outside of a main body 1b; a blood circuit 3 which is connected to the dialyzer 2; a dialysate circuit 4 which is connected to the dialyzer 2 and also is provided in the main body 1b; a replenishing liquid passage 5 which is connected between the blood circuit 3 and the dialysate circuit 4; and an auxiliary power source 6 which supplies an electric power to a part of the hemodialysis apparatus at the time of the power failure.

In addition, the control means 1a has a screen display type operation panel lc, a button, an icon and a message which are necessary for the operation are displayed on the screen, and the control means 1a can set the operation and various parameters of the apparatus.

The hemodialysis apparatus 1 of the present example is a hemodialysis apparatus 1 that can be applied to a personal ON-LINE HDF which performs a hemo-diafiltration therapy (HDF) for a patient, and can perform priming when the hemodialysis apparatus 1 starts its operation, liquid replenishment in a dialysis treatment and retransfusion when the dialysis treatment is finished, by sending the dialysate using the replenishing liquid passage 5.

The dialyzer 2 includes an outer case 2a made from a resin and a large number of hollow fibers 2b which constitute a filtration film provided in the outer case 2a. The inner part of the hollow fiber 2b communicates with the blood circuit 3, and the blood passes through the inner part. A space between the outer case 2a and the hollow fiber 2b communicates with the dialysate circuit 4, and the dialysate passes through the space in an opposite direction to the flow of the blood.

The auxiliary power source 6 is provided in the inner part of the main body portion 1b, is used as an internal power source such as a battery, and supplies an electric power to a part of the equipment of the hemodialysis apparatus 1 when an electric power is not supplied from the outlet C due to the power failure.

The blood circuit 3 includes the artery side passage 11 which is connected to the blood vessel of the patient and supplies the blood to the dialyzer 2 therethrough and the vein side passage 12 for returning the blood to the patient from the dialyzer 2 therethrough. These passages are formed of a tube made from silicone.

In the artery side passage 11, a puncture needle 11a with which the blood vessel of the patient is punctured is provided in one end, and also the dialyzer 2 is connected to the other end; and a clamp 13 for closing the artery side passage 11, a blood pump 14 for sending the blood and a drip chamber 15 are arranged sequentially from the puncture needle 11a. The drip chamber 15 is provided with a pressure gauge 15a.

The blood pump 14 is a roller pump which sends the blood by wringing a tube, and also is driven by the auxiliary power source 6. The operation is controlled by the control means 1a, and the blood pump 14 is enabled to send the blood from the patient to the dialyzer 2.

The vein side passage 12 has one end connected to the dialyzer 2, also has the other end provided with a puncture needle 12a with which the blood vessel of the patient is punctured, and has a drip chamber 16 and a clamp 17 for closing the vein side passage 12 arranged sequentially from the dialyzer 2. The drip chamber 16 is provided with a pressure gauge 16a.

The dialysate circuit 4 includes a dialysate supply passage 23 for supplying a fresh dialysate from a first dialysate chamber 21 or a second dialysate chamber 22 to the dialyzer 2 therethrough and a dialysate recovery passage 24 for recovering a used dialysate therethrough which has passed the dialyzer 2 to the first dialysate chamber 21 or the second dialysate chamber 22. These passages are formed of a tube made from silicone.

The first dialysate chamber 21 and the second dialysate chamber 22 have the same shape. Their inner parts are divided into two chambers by diaphragms, respectively. The chambers in one side are named as supply chambers 21a and 22a for producing and supplying the fresh dialysate, and the chambers in the other side are named as recovery chambers 21b and 22b for recovering the used dialysate.

The dialysate supply passage 23 is branched and connected to the supply chambers 21a and 22a of the first and second dialysate chambers 21 and 22, respectively, and the dialysate recovery passage 24 is also branched and connected to the recovery chambers 21b and 22b.

In addition, a water supply passage 25 for supplying purified water therethrough is connected to the supply chambers 21a and 22a of the first and second dialysate chambers 21 and 22, respectively, and a drainage passage 26 for discharging the used dialysate therethrough is connected to the recovery chambers 21b and 22b.

The water supply passage 25 has an upstream portion connected to not-shown water supply means which supplies purified water such as RO water, and a downstream portion which is branched out into two directions and connected to the supply chambers 21a and 22a of the first and second dialysate chambers 21 and 22, respectively. The supply chambers 21a and 22a are provided with water supply valves V1 and V2, respectively, which are opened and closed by the control of the control means 1a.

In the water supply passage 25, a purified water pump 31 for sending the purified water is provided. An A liquid supply passage 32 for supplying an A liquid therethrough which is a stock solution of the dialysate and a B liquid supply passage 33 for supplying a B liquid therethrough which is a stock solution of the dialysate are connected to each point between this purified water pump 31 and a bifurcation area.

An A liquid container 34 and a B liquid container 35 are structured so as to be connected to the upstream portions of the A liquid supply passage 32 and the B liquid supply passage 33, respectively, and the A liquid pump 36 provided in the A liquid supply passage 32 and the B liquid pump 37 provided in the B liquid supply passage 33 are structured so as to send the A liquid and the B liquid therethrough, respectively.

As for the dialysate supply passage 23, the upstream portion thereof is branched out into two directions, and the branched portions are connected to the supply chambers 21a and 22a of the first and second dialysate chambers 21 and 22, respectively; and the downstream portion thereof is connected to the dialyzer 2. Supply valves V3 and V4 which are opened and closed by the control of the control means 1a are provided in the branched portions, respectively.

In addition, in the dialysate supply passage 23, a concentration sensor 41 for measuring the concentration of the dialysate, a first dialysate filter F1 for removing a harmful component of the dialysate, and a first open/close valve V5 which is opened/closed by the control of the control means 1a are provided sequentially from the branch point.

A first bypass passage 42, which connects the dialysate supply passage 23 with the dialysate recovery passage 24 to communicate with each other, is connected to the primary side of the first dialysate filter F1, and a second open/close valve V6 which is opened/closed by the control of the control means 1a is provided in the first bypass passage 42.

When the second open/close valve V6 is opened, the dialysate in the dialysate supply passage 23 can be sent to the dialysate recovery passage 24 through the first bypass passage 42 without passing through the dialyzer 2, and for instance, the dialysate can be discharged of which the concentration has been detected to be incorrect by the concentration sensor 41.

In addition, in the dialysate supply passage 23, opening means 43 for opening the sealed dialysate circuit 4 to the outside is provided between the first dialysate filter F1 and the first open/close valve V5.

The opening means 43 includes a third open/close valve V7 which is opened/closed by the control of the control means la, a check valve 45 for preventing the outflow of the dialysate and an air filter 46 for purifying the influent air, which are provided in an opening passage 44 connected between the first dialysate filter F1 and the first open/close valve V5 in the dialysate supply passage 23.

The third open/close valve V7 is structured so as to be operated by the auxiliary power source 6, and is set so as to be opened in an auxiliary power source retransfusing mode which will be described later.

When the third open/close valve V7 is opened, the inflow of the outside air to the dialysate circuit 4 constituting a closing circuit is permitted, and a negative pressure in the dialysate circuit 4 can be opened to the outside pressure. In addition, the air which has flowed into the dialysate circuit 4 at this time is purified by the air filter 46, and the check valve 45 is structured so as to prevent the outflow of the dialysate.

Furthermore, the dialysate supply passage 23 includes a branch passage 47 which is branched between the first open/close valve V5 and the dialyzer 2, and the downstream portion of this branch passage 47 is connected to the dialysate recovery passage 24.

In the branch passage 47, a storage container 48 for storing the dialysate therein, a second dialysate filter F2 for removing a harmful component of the dialysate, and a fourth open/close valve V8 which is opened/closed by the control of the control means 1a are provided, and a connection port 49 for connecting the replenishing liquid passage 5 thereto is provided between the second dialysate filter F2 and the fourth open/close valve V8.

The storage container 48 is structured so as to store a fresh dialysate therein which passes the dialysate supply passage 23, and is determined so as to store a dialysate necessary for the blood in the blood circuit 3 to be retransfused into the patient, which will be described later.

The fourth open/close valve V8 is usually closed, and the dialysate which passes through the branch passage 47 is sent to the replenishing liquid passage 5 through the connection port 49. The fourth open/close valve V8 is structured so as to be opened, for instance, in a priming or cleaning operation, and send the dialysate in the dialysate supply passage 23 to the dialysate recovery passage 24.

A second bypass passage 50, which connects the dialysate supply passage 23 with the dialysate recovery passage 24 to communicate with each other, is connected to the primary side of the second dialysate filter F2, and a fifth open/close valve V9 which is opened/closed by the control of the control means 1a is provided in the second bypass passage 50.

When the fifth open/close valve V9 is opened, the dialysate in the dialysate supply passage 23 can be sent to the dialysate recovery passage 24 through the second bypass passage 50, without being passed through the dialyzer 2.

As for the dialysate recovery passage 24, the upstream portion thereof is connected to the dialyzer 2, and the downstream portion thereof is branched into two directions. The branched portions are connected to the recovery chambers 21b and 22b of the first and second dialysate chambers 21 and 22, respectively, and recovery valves V10 and V11 which are opened and closed by the control of the control means 1a are provided in these branched portions, respectively.

In addition, in the dialysate recovery passage 24, a pressure sensor 51 for measuring the pressure in the dialysate circuit 4 and a dialysate pump 52 for sending the dialysate are sequentially provided from the dialyzer 2.

As for the drainage passage 26, the upstream portion thereof is branched out into two directions, and the branched portions are connected to the recovery chambers 21b and 22b of the first and second dialysate chambers 21 and 22, respectively; and the downstream portion thereof is connected to a not-shown drain pipe installed in a medical institution. Drain valves V12 and V13 which are opened and closed by the control of the control means 1a are provided in the branched portions, respectively.

The water removing passage 53 is connected between the dialysate recovery passage 24 and the drainage passage 26. The upstream portion thereof is connected between the dialysate pump 52 in the dialysate recovery passage 24 and the branch point, and the downstream portion thereof is connected to the downstream side of the branch point in the drainage passage 26.

A water removing pump 54 for discharging the used dialysate from the dialysate recovery passage 24 and subjecting the used dialysate to ultrafiltration is provided in the water removing passage 53.

According to the dialysate circuit 4 having the above described structure, when the fresh dialysate is produced in the first dialysate chamber 21, the control means 1a opens the water supply valve V1 and the drain valve V12 and simultaneously closes the supply valve V3 and the recovery valve V10.

Then, because the water supply valve V1 and the drain valve V12 are opened in the first dialysate chamber 21, the purified water, the stock solution A and the stock solution B flow into the supply chamber 21a from the water supply passage 25, and these solutions are mixed there to produce the fresh dialysate. On the other hand, in the recovery chamber 21b, a diaphragm is pressed and the used dialysate is discharged to the drainage passage 26.

On the other hand, in the second dialysate chamber 22, the control means 1a opens the supply valve V4 and the recovery valve V11, and also closes the water supply valve V2 and the drain valve V13. Then, the used dialysate which has been sent by the dialysate pump 52 flows into the recovery chamber 22b, and thereby the produced fresh dialysate results in being supplied into the dialysate supply passage 23 from the supply chamber 22a.

After that, the control means 1a alternately opens and closes the water supply valves V1 and V2, the supply valves V3 and V4, the recovery valves V10 and V11, and the drain valves V12 and V13. Thereby, the fresh dialysate which has been produced in the first dialysate chamber 21 and the second dialysate chamber 22 results in being supplied to the dialyzer 2 through the dialysate supply passage 23, and the used dialysate which has passed through the dialyzer 2 results in being discharged through the drainage passage 26.

The replenishing liquid passage 5 is formed of a tube made from silicone. The upstream portion thereof is connected to the connection port 49 provided in the branch passage 47 which constitutes the dialysate supply passage 23, and the downstream portion thereof is connected between the end portion which is connected to the patient in the artery side passage 11 and the blood pump 14.

In addition, a replenishing liquid pump 61 of which the operation is controlled by the control means 1a is arranged in the replenishing liquid passage 5, and is structured to be driven also by the auxiliary power source 6 in an auxiliary power source retransfusing mode which will be described later, similarly to the blood pump 14.

The replenishing liquid pump 61 is a roller pump which sends the blood by wringing the tube, and is structured so as to send the fresh dialysate from the dialysate supply passage 23 to the blood circuit 3.

Incidentally, a pillow 62 which has a larger cross section of the flow channel than that of a tube which constitutes the replenishing liquid passage 5 as pressure detection means, is formed into a flat shape and is made from a resin is provided between a connection portion with the artery side passage 11 in the replenishing liquid passage 5 and the replenishing liquid pump 61.

The operation of the hemodialysis apparatus 1 having the above described structure will be described below.

Firstly, in a priming operation before the treatment, the hemodialysis apparatus produces the dialysate in a similar way to that during the treatment, fills the dialysate circuit 4 with the dialysate, further operates the replenishing liquid pump 61, passes the fresh dialysate to the blood circuit 3, and fills the blood circuit 3 and the dialyzer 2 with the dialysate. At this time, the storage container 48 provided in the replenishing liquid passage 5 is also turned into a state of being filled with the fresh dialysate.

When this priming operation has been finished, an operator connects the puncture needle 11a of the artery side passage 11 and the puncture needle 12a of the vein side passage 12 to the blood vessels of the patient, respectively. After that, the control means 1a operates the blood pump 14 to extract the blood from the patient and pass the blood to the dialyzer 2. At this time, the operator observes the pillow 62 provided in the replenishing liquid passage 5 and observes whether the blood smoothly flows into the artery side passage 11 or not.

When the blood does not smoothly flow into the dialyzer 2 by the reason that the puncture needle 11a is clogged or the like, the upstream side of the blood pump 14 becomes a negative pressure, and the downstream side of the replenishing liquid pump 61 in the replenishing liquid passage 5 also becomes the negative pressure, because the control means 1a stops the replenishing liquid pump 61. Accordingly, the pillow 62 is recessed and the operator can visually check the failure of blood extraction.

For information, in a conventional hemodialysis apparatus 1, a similar pillow is provided in the artery side passage 11 and the blood passes through the inner part of the artery side passage 11. Accordingly, a thrombus might occur in a portion at which the flow stagnates in the pillow.

In contrast to this, in the present example, the pillow 62 is provided in the replenishing liquid passage 5. Because of this, the blood does not pass through the inner part of the pillow 62, and accordingly the thrombus does not occur.

When the blood is placed in a state of passing through the blood circuit 3 by the blood extraction operation, the priming operation is switched to a dialysis operation. When the replenishing liquid pump 61 is operated in the dialysis treatment, a part of the fresh dialysate of the dialysate supply passage 23 is supplied to the artery side passage 11 through the replenishing liquid passage 5, and the fresh dialysate is enabled to be replenished to the patient.

Here, the dialysate to be used for this replenishing liquid is a fresh dialysate which has been produced in the first and second dialysate chambers 21 and 22. In addition, the fresh dialysate becomes a safe dialysate in which endotoxin is removed, because of having passed through the first and second dialysate filters F1 and F2 provided in the dialysate supply passage 23.

Furthermore, by the replenishing liquid pump 61 which has been operated, the fresh dialysate flows into the storage passage 48 provided in the branch passage 47, and the storage passage 48 is placed in a state in which a necessary amount of the fresh dialysate is always stored in the storage passage 48.

When the dialysis treatment has been finished, the dialysis treatment is switched to a retransfusion operation for returning the blood having remained in the blood circuit 3 to the patient. At this time, the supply of the fresh dialysate from the first and second dialysate chambers 21 and 22 is continued from the beginning of the dialysis treatment on.

The control means 1a stops the blood pump 14 and then operates the replenishing liquid pump 61. Because the artery side passage 11 is blocked at a position of the blood pump 14, the dialysate supplied from the replenishing liquid passage 5 flows toward the patient in an opposite direction to the direction of the blood which has been sent during the dialysis treatment, and the blood remaining in the artery side passage 11 is retransfused into the patient.

When the retransfusion of the blood from the artery side passage 11 has been finished, the control means 1a closes the artery side passage 11 by the clamp 13 and simultaneously operates the blood pump 14.

Thereby, the dialysate supplied from the replenishing liquid passage 5 is passed in the same direction as the direction of the blood which is sent during the dialysis treatment, passes through the hollow fiber 2b of the dialyzer 2 and is sent to the vein side passage 12. Then, the blood having remained in the artery side passage 11, the vein side passage 12 and the dialyzer 2 is retransfused into the patient through the vein side passage 12.

In the above description, the operation according to a normal operation to be conducted when an electric power is supplied from an outlet C has been described, but in the next place, the case will be described in which the power failure has occurred during the dialysis treatment.

Specifically, when the electric power becomes incapable of being supplied from the outlet C due to the power failure in the above described dialysis treatment, the dialysis operation is stopped, and the dialysate becomes incapable of being produced and supplied.

When the power failure has occurred and an alarm sounds, the operator commands the auxiliary power source retransfusing mode to the control means 1a through the operation panel lc provided on the control means 1a. Incidentally, the control means 1a receives the supply of the electric power from the auxiliary power source 6, and keeps an acting state even after the power failure.

Thereby, the control means 1a makes the opening means 43 operate by the auxiliary power source 6, open the third open/close valve V7, and turn the dialysate circuit 4 into a state of being opened to the outside through the opening passage 44.

Furthermore, the control means 1a makes the replenishing liquid pump 61 operate by the electric power of the auxiliary power source 6 in such a state that the blood pump 14 is stopped, and send the fresh dialysate stored in the storage container 48 provided in the branch passage 47 to the blood circuit 3 through the replenishing liquid passage 5.

Thereby, the dialysate passes through the artery side passage 11 in an opposite direction to the direction of the blood which has been sent in the dialysis treatment, and the blood is retransfused into the patient from the artery side passage 11 by the same operation as that in the normal operation.

At this time, the dialysate circuit 4 which is structured so as to act as a closed circuit in the normal state due to the opening means 43 is opened to the air. Accordingly, the dialysate circuit 4 is structured so that even when the replenishing liquid pump 61 is operated in a state in which the supply of the dialysate from the first dialysate chamber 21 and the second dialysate chamber is stopped, its inner part does not become a negative pressure and the dialysate is not hindered from being sent.

Incidentally, the specific capacity of the storage container 48 may be set so as to be a sufficient amount of being capable of retransfusing all of the blood in the blood circuit 3, in consideration of the capacity of the dialysate remaining in the dialysate supply passage 23 and the replenishing liquid passage 5.

When the retransfusion of the blood from the artery side passage 11 has been finished, the control means 1a makes the clamp 13 operate by the auxiliary power source 6 and close the artery side passage 11, and makes also the blood pump 14 operate. Thereby, the dialysate which has been supplied from the replenishing liquid passage 5 passes from the artery side passage 11 to the vein side passage 12 through the dialyzer 2, and the blood is retransfused into the patient from the vein side passage 12 by the same operation as that in the normal operation.

As described above, the hemodialysis apparatus 1 in the above described example is structured so as to be capable of performing retransfusion due to the auxiliary power source 6, by setting the control of the control means 1a to the auxiliary power source retransfusing mode, even when having become incapable of deriving an electric power from the outlet C during the dialysis treatment due to the power failure.

The hemodialysis apparatus 1 is also structured so as to be capable of retransfusing all of the blood in the blood circuit 3 into the patient even in a state in which the supply of the new dialysate is stopped, because of having the storage container 48 provided in the branch passage 47 and retransfusing the blood in the blood circuit 3 into the patient by using the dialysate stored in the storage container 48.

The hemodialysis apparatus 1 is structured so as to prevent the dialysate at this time from being hindered from being sent to the replenishing liquid passage 5 due to a phenomenon that the inner part of the dialysate circuit 4 becomes a negative pressure by the operation of the replenishing liquid pump 61, by opening the dialysate circuit 4 which is a sealed closed circuit to the outside by the opening means 43.

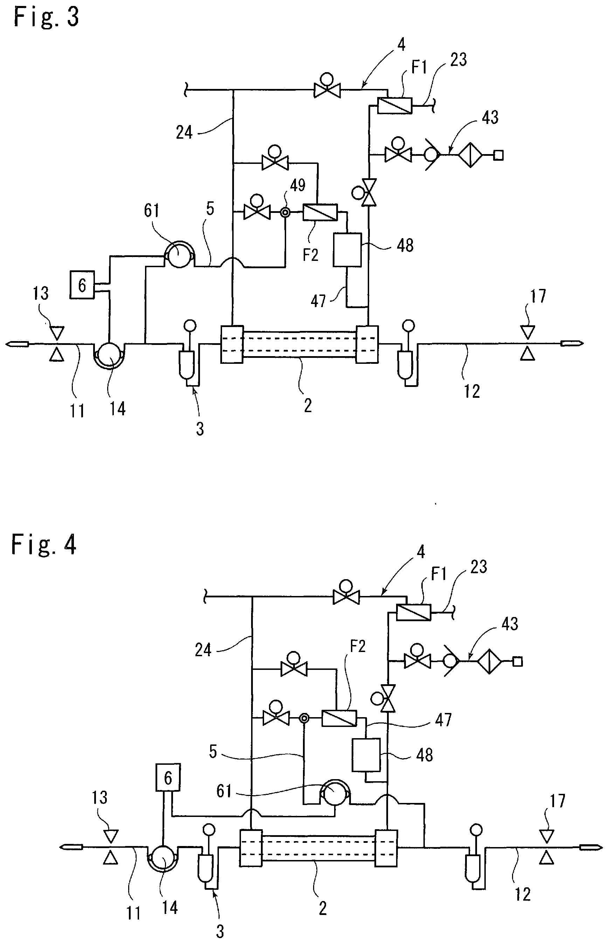

Next, Figure 3 is a view for describing a part of the liquid circuit of a hemodialysis apparatus 1 according to a second embodiment. The present example is different from the first example only in a point that the connection position of the replenishing liquid passage 5 is different from that in the first embodiment as will be described below. Accordingly, the detailed description of the structure except the connection position will be omitted, and the present example will be described while using the reference numerals which have been used in the first example as they are.

In the second embodiment, the downstream portion in the replenishing liquid passage 5 is structured so as to be connected between the blood pump 14 and the dialyzer 2 in the artery side passage 11, and the blood is retransfused in the normal retransfusion operation and the auxiliary power source retransfusing mode, in the following way.

Firstly, in order to retransfuse the blood in the blood circuit 3 from the artery side passage 11 into the patient, the control means 1a operates the replenishing liquid pump 61 and operates also the blood pump 14 so as to send the blood in a direction opposite to the direction taken when the blood is sent in the dialysis treatment. At this time, the clamp 17 is closed so that the blood is not drawn from the patient.

Thereby, the blood having remained in a more upstream side (end portion side to be connected to patient) than the connection position of the replenishing liquid passage 5 in the artery side passage 11 results in being retransfused into the patient from the artery side passage 11 by the action of the fresh dialysate which has been supplied from the replenishing liquid passage 5 and is passed in an opposite direction to the direction of the blood which has been sent in the dialysis treatment.

The control means 1a stops the blood pump 14 in a state of having operated the replenishing liquid pump 61, blocks the artery side passage 11, opens the clamp 17, and passes the dialysate from the artery side passage 11 to the vein side passage 12.

Thereby, the blood having remained in a more downstream side (end portion side to be connected to dialyzer 2) than the connection position of the replenishing liquid passage 5 in the artery side passage 11 and having remained in the vein side passage 12 is retransfused into the patient through the vein side passage 12 by the action of the fresh dialysate which has been supplied from the replenishing liquid passage 5 and is passed from the artery side passage 11 to the vein side passage 12 through the dialyzer 2.

In this second embodiment as well, in the auxiliary power source retransfusing mode, the opening means 43 is structured so as to be operated by the auxiliary power source 6 to open the dialysate circuit 4 to the outside, and the replenishing liquid pump 61, the blood pump 14 and the clamp 17 are structured so as to operate by the auxiliary power source 6, in a similar way to that in the first example.

Figure 4 is a view for describing a part of the liquid circuit of a hemodialysis apparatus 1 according to a third embodiment. The present example is different from the first and second embodiments only in a point that the connection position of the replenishing liquid passage 5 is different from those in the first and second embodiments as will be described below. Accordingly, the detailed description of the structure except the connection position will be omitted, and the present example will be described while using the reference numerals which have been used in the first example as they are.

In the third embodiment, the downstream portion in the replenishing liquid passage 5 is structured so as to be connected to the vein side passage 12, and the blood is retransfused in the normal retransfusion operation and the auxiliary power source retransfusing mode, in the following way.

Firstly, in order to retransfuse the blood in the blood circuit 3 from the artery side passage 11 into the patient, the control means 1a operates the replenishing liquid pump 61 and operates also the blood pump 14 so as to send the blood in a direction opposite to the direction taken when the blood is sent in the dialysis treatment. At this time, the clamp 17 is closed so that the blood is not drawn from the patient.

Thereby, the blood having remained in a more upstream side (end portion side to be connected to dialyzer 2) than the connection position of the replenishing liquid passage 5 in the vein side passage 12 and having remained in the artery side passage 11 results in being retransfused into the patient from the artery side passage 11 by the action of the fresh dialysate which has been supplied from the replenishing liquid passage 5 and is passed in an opposite direction to the direction of the blood which has been sent in the dialysis treatment.

The control means 1a stops the blood pump 14 in a state of having operated the replenishing liquid pump 61, blocks the artery side passage 11, opens the clamp 17, and passes the dialysate to the vein side passage 12.

Thereby, the blood having remained in a more downstream side (end portion side to be connected to patient) than the connection position of the replenishing liquid passage 5 in the vein side passage 12 results in being retransfused into the patient from the vein side passage 12 by the action of the fresh dialysate which has been supplied from the replenishing liquid passage 5 and is passed in the same direction as the direction of the blood that has been sent in the dialysis treatment.

As in the second and third embodiments, even when the connection position in the downstream portion of the replenishing liquid passage 5 is changed, the hemodialysis apparatus 1 is enabled to retransfuse the blood due to the auxiliary power source 6, by setting the auxiliary power source retransfusing mode in the control means 1a when the power failure has occurred in the dialysis treatment, in a similar way to that in the first example.

Incidentally, the opening means 43 in the above described example can be provided not on the dialysate supply passage 23 but on the dialysate recovery passage 24 instead, and can be structured so as to open various portions in the dialysate circuit 4 except for portions between the storage container 48 and the replenishing liquid pump 61.

For instance, the opening means 43 is provided in the side closer to the dialyzer 2 than the dialysate pump 52 in the dialysate recovery passage 24, and the second open/close valve V6 provided in the first bypass passage 42 is enabled to be opened/closed by the auxiliary power source 6. Then, the dialysate circuit 4 which is a closed circuit can be opened to the outside, and the dialysate in the storage container 48 can be sent to the replenishing liquid passage 5 by the replenishing liquid pump 61 in a state in which the supply of the dialysate from the first and second dialysate chambers 21 and 22 is stopped, when the hemodialysis apparatus 1 is set to the auxiliary power source retransfusing mode.

Furthermore, the opening means 43 may also be determined to be drain valves V12 and V13 which are driven by the auxiliary power source 6 and are provided in the drainage passage 26, which is different from the above described structure. Then, the dialysate circuit 4 can be opened to the outside by opening these drain valves V12 and V13.

In addition, the storage container 48 can be also provided in the more upstream side than the connection portion of the branch passage 47. However, when the structure is adopted in a personal hemodialysis apparatus, the dialysate having a correct concentration may not be produced due to the power failure. In order to prevent the dialysate having an incorrect concentration from being supplied to the blood circuit 3, the storage container 48 is desirably provided in a more downstream side than the concentration sensor 41 and stores the dialysate having the correct concentration therein. In addition, the storage container 48 can be provided also in the replenishing liquid passage 5 between the connection port 49 and the replenishing liquid pump 61.

Furthermore, the hemodialysis apparatus 1 in the above described example has been described as a personal hemodialysis apparatus, but may also be a monitoring apparatus for dialysis, which performs blood dialysis by using a dialysate to be supplied from a dialysate supply apparatus.

### Reference Signs List

- 1: Hemodialysis apparatus
- 2: Dialyzer
- 3: Blood circuit
- 4: Dialysate circuit
- 5: Replenishing liquid passage
- 6: Auxiliary power source
- 11: Artery side passage
- 12: Vein side passage
- 14: Blood pump
- 23: Dialysate supply passage
- 24: Dialysate recovery passage
- 43: Opening means
- 48: Storage container
- 61: Replenishing liquid pump
- 62: Pillow
- F1: First dialysate filter
- F2: Second dialysate filter

## Claims

1. A hemodialysis apparatus (1) that is provided with a dialyzer (2) which performs hemodialysis, a dialysate circuit (4) which comprises a dialysate supply passage (23) for supplying a fresh dialysate to the dialyzer (2) therethrough and a dialysate recovery passage (24) for recovering a used dialysate therethrough which has passed the dialyzer (2), a blood circuit (3) which comprises an artery side passage (11) for sending blood to the dialyzer (2) from a patient therethrough and a vein side passage (12) for returning the blood to the patient from the dialyzer (2) therethrough, a replenishing liquid passage (5) which connects the dialysate supply passage (23) with the blood circuit (3) to communicate with each other, a replenishing liquid pump (61) which is arranged in the replenishing liquid passage (5) and sends the dialysate from the dialysate supply passage (23) to the blood circuit (3), a dialysate filter (F1) which is provided in the dialysate supply passage (23) and purifies the dialysate, a blood pump (14) which is arranged in the artery side passage (11), and control means (1a) which controls the operation of the replenishing liquid pump (61) and the blood pump (14); and the control means (1a) is configured to, when the blood in the blood circuit (3) is retransfused into the patient, operate the replenishing liquid pump (61) to pass a fresh dialysate, which has been purified by the dialysate filter (F1), to the blood circuit (3) through the replenishing liquid passage (23) to retransfuse the blood into the patient by the dialysate, wherein
the hemodialysis apparatus further comprises opening means (43) which opens the sealed dialysate circuit (4) to the outside, a storage container (48) which stores therein the fresh dialysate passing through the dialysate supply passage (23), and an auxiliary power source (6) for operating at least the replenishing liquid pump (61), the blood pump (14), the opening means (43) and the control means (1a);
**characterized in that**
an auxiliary power source (6) retransfusing mode is set in the control means (1a) as to operate the replenishing liquid pump (61), the blood pump (44) and the opening means (43) by the auxiliary power source (6), send the fresh dialysate stored in the storage container (48) to the blood circuit (3) through the replenishing liquid passage (23), and retransfuse the blood into the patient.

2. The hemodialysis apparatus (1) according to claim 1, wherein
the replenishing liquid passage (23) is connected between the end of the artery side passage (11) connected with the patient and the blood pump (14);
the control means (1a) is configured to stop the blood pump (14) when the blood in the blood circuit (3) is retransfused from the artery side passage (11) into the patient, and is configured to operate the replenishing liquid pump (61) to pass the dialysate to the artery side passage (11) in an opposite direction to the direction of the blood which has been sent; and
the control means (1a) is configured to operate the replenishing liquid pump (61) and the blood pump (14) when the blood is retransfused into the patient from the vein side passage (12), and to pass the dialysate from the artery side passage (11) to the vein side passage (12).

3. The hemodialysis apparatus (1) according to claim 2, further comprising pressure detection means which is provided between a connection portion between the artery side passage (11) and the replenishing liquid passage (5), and the replenishing liquid pump (61).

4. The hemodialysis apparatus according to claim 1, wherein
the replenishing liquid passage (5) is connected between the blood pump (14) in the artery side passage (11) and the dialyzer (2);
the control means (1a) is configured to operate the replenishing liquid pump (61) when the blood in the blood circuit (3) is retransfused from the artery side passage (11) into the patient, and is configured to operate the blood pump (14) so as to send the blood in a direction opposite to the direction taken when the blood is sent, and to pass the dialysate to the artery side passage (11) in an opposite direction to the direction of the blood which has been sent; and
the control means (1a) is configured to operate the replenishing liquid pump (61) when the blood is retransfused from the vein side passage (12) into the patient, and to pass the dialysate from the artery side passage (11) to the vein side passage (11).

5. The hemodialysis apparatus (1) according to claim 1, wherein
the replenishing liquid passage (5) is connected with the vein side passage (12);
the control means (1a) is configured to operate the replenishing liquid pump (61) when the blood in the blood circuit (3) is retransfused from the artery side passage (12) into the patient, and is also configured to operate the blood pump (14) so as to send the blood in a direction opposite to the direction taken when the blood is sent, and to pass the dialysate from the vein side passage (12) to the artery side passage (11) in an opposite direction to the direction of the blood which has been sent; and
the control means (1a) is configured to operate the replenishing liquid pump (61) when the blood is retransfused from the vein side passage (12)into the patient, and to pass the dialysate to the vein side passage (12).

## Patentansprüche

1. Hämodialyse-Vorrichtung (1), die mit einem Dialysator (2), der die Hämodialyse durchführt, einem Dialysatkreislauf (4), der einen Dialysatzufuhrdurchlass (23) zum Zuführen von frischem Dialysat durch diesen hindurch an den Dialysator (2) und einen Dialysatrückführdurchlass (24) zum Rückführen eines verwendeten Dialysats durch diesen hindurch, das durch den Dialysator (2) geführt wurde, umfasst, einem Blutkreislauf (3), der einen Arterienseitendurchlass (11) zum Leiten von Blut aus einem Patienten durch diesen hindurch an den Dialysator (2) und einen Venenseitendurchlass (12) zum Rückführen des Bluts an den Patienten durch diesen hindurch von dem Dialysator (2), umfasst, einem Flüssigkeitsauffülldurchlass (5), der den Dialysatzufuhrdurchlass (23) zur wechselseitigen Kommunikation mit dem Blutkreislauf (3) verbindet, einer Flüssigkeitsauffüllpumpe (61), die in dem Flüssigkeitsauffülldurchlass (5) angeordnet ist und das Dialysat von dem Dialysatzufuhrdurchlass (23) an den Blutkreislauf (3) weiterführt, einem Dialysatfilter (F1), der in dem Dialysatzufuhrdurchlass (23) vorgesehen ist und das Dialysat reinigt, einer Blutpumpe (14), die in dem Arterienseitendurchlass (11) angeordnet ist, und einem Steuermittel (1a) versehen ist, das den Betrieb der Flüssigkeitsauffüllpumpe (61) und der Blutpumpe (14) steuert; und
wobei das Steuermittel (1a) konfiguriert ist, wenn das Blut in dem Blutkreislauf (3) in den Patienten rücktransfundiert wird, die Flüssigkeitsauffüllpumpe (61) so zu betreiben, dass frisches Dialysat, das von dem Dialysatfilter (F1) gereinigt wurde, durch den Flüssigkeitsauffülldurchlass (23) an den Blutkreislauf (3) weiterzuführen, um das Blut über das Dialysat an den Patienten zurück zu transfundieren, wobei
die Hämodialyse-Vorrichtung ferner ein Öffnungsmittel (43), das den abgedichteten Dialysatkreislauf (4) nach außen hin öffnet, einen Sammelbehälter (48), in dem das frische Dialysat, das durch den Dialysatzufuhrdurchlass (23) geführt wird, gesammelt wird, und eine Hilfsleistungsquelle (6) zum Betreiben von zumindest der Flüssigkeitsauffüllpumpe (61), der Blutpumpe (14), dem Öffnungsmittel (43) und dem Steuermittel (1a) umfasst;
**dadurch gekennzeichnet, dass**:
ein Rücktransfusionsmodus der Hilfsleistungsquelle (6) in dem Steuermittel (1a) zum Betreiben der Flüssigkeitsauffüllpumpe (61), der Blutpumpe (44) und des Öffnungsmittels (43) durch die Hilfsleistungsquelle (6) eingestellt ist, um frisches Dialysat, das in dem Sammelbehälter (48) gesammelt ist, durch den Flüssigkeitsauffülldurchlass (23) an den Blutkreislauf (3) weiterzuführen und das Blut in den Patienten zurück zu transfundieren.

2. Hämodialyse-Vorrichtung (1) nach Anspruch 1,
wobei
der Flüssigkeitsauffülldurchlass (23) zwischen das Ende des Arterienseitendurchlasses (11), der mit dem Patienten verbunden ist, und die Blutpumpe (14), geschaltet ist;
das Steuermittel (1a) konfiguriert ist, die Blutpumpe (14) anzuhalten, wenn das Blut in dem Blutkreislauf (3) von dem Arterienseitendurchlass (11) in den Patienten rücktransfundiert wird, und konfiguriert ist, die Flüssigkeitsauffüllpumpe (61) zu betreiben, um das Dialysat in eine der Richtung des weitergeführten Bluts entgegengesetzten Richtung an den Arterienseitendurchlass (11) weiterzuführen; und
das Steuermittel (1a) konfiguriert ist, die Flüssigkeitsauffüllpumpe (61) und die Blutpumpe (14) zu betreiben, wenn das Blut von dem Venenseitendurchlass (12) an den Patienten rücktransfundiert wird, und das Dialysat von dem Arterienseitendurchlass (11) an den Venenseitendurchlass (12) weiterzuleiten.

3. Hämodialyse-Vorrichtung (1) nach Anspruch 2, ferner umfassend ein Druckdetektionsmittel, das zwischen einem Verbindungsabschnitt zwischen dem Arterienseitendurchlass (11) und dem Flüssigkeitsauffülldurchlass (5) und der Flüssigkeitsauffüllpumpe (61) bereitgestellt ist.

4. Hämodialyse-Vorrichtung nach Anspruch 1, wobei der Flüssigkeitsauffülldurchlass (5) zwischen die Blutpumpe (14) in dem Arterienseitendurchlass (11) und den Dialysator (2) geschaltet ist;
das Steuermittel (1a) konfiguriert ist, die Flüssigkeitsauffüllpumpe (61) zu betreiben, wenn das Blut in dem Blutkreislauf (3) von dem Arterienseitendurchlass (11) in den Patienten rücktransfundiert wird, und konfiguriert ist, die Blutpumpe (14) so zu betreiben, um das Blut in eine Richtung weiterzuleiten, die entgegengesetzt zu jener Richtung ist, die zum Weiterleiten des Bluts verwendet wurde, und das Dialysat in eine Richtung an den Arterienseitendurchlass (11) weiterzuführen, die entgegengesetzt zu jener Richtung ist, die zum Weiterleiten des Bluts verwendet wurde;
und
das Steuermittel (1a) konfiguriert ist, die Flüssigkeitsauffüllpumpe (61) zu betreiben, wenn das Blut von dem Venenseitendurchlass (12) in den Patienten rücktransfundiert wird, und das Dialysat von dem Arterienseitendurchlass (11) an den Venenseitendurchlass (11) weiterzuführen.

5. Hämodialyse-Vorrichtung (1) nach Anspruch 1,
wobei
der Flüssigkeitsauffülldurchlass (5) mit dem Venenseitendurchlass (12) verbunden ist;
das Steuermittel (1a) konfiguriert ist, die Flüssigkeitsauffüllpumpe (61) zu betreiben, wenn das Blut in dem Blutkreislauf (3) von dem Arterienseitendurchlass (12) in den Patienten rücktransfundiert wird, und ebenfalls konfiguriert ist, die Blutpumpe (14) so zu betreiben, um das Blut in eine Richtung weiterzuleiten, die entgegengesetzt zu jener Richtung ist, die zum Weiterleiten des Bluts verwendet wurde, und das Dialysat von dem Venenseitendurchlass (12) in eine Richtung an den Arterienseitendurchlass (11) weiterzuführen, die entgegengesetzt zu jener Richtung ist, die zum Weiterleiten des Bluts verwendet wurde; und
das Steuermittel (1a) konfiguriert ist, die Flüssigkeitsauffüllpumpe (61) zu betreiben, wenn das Blut von dem Venenseitendurchlass (12) in den Patienten rücktransfundiert wird, und das Dialysat an den Venenseitendurchlass (12) weiterzuführen.

## Revendications

1. Appareil d'hémodialyse (1) qui est doté d'un dialyseur (2) qui réalise l'hémodialyse, d'un circuit de dialysat (4) qui comprend un passage d'alimentation en dialysat (23) pour fournir un dialysat frais au dialyseur (2) à travers lui et un passage de récupération de dialysat (24) pour récupérer un dialysat usagé à travers lui qui a traversé le dialyseur (2), d'un circuit sanguin (3) qui comprend un passage côté artère (11) pour envoyer le sang au dialyseur (2) d'un patient à travers lui et un passage côté veine (12) pour renvoyer le sang au patient depuis le dialyseur (2) à travers lui, d'un passage de liquide de réapprovisionnement (5) qui relie le passage d'alimentation en dialysat (23) au circuit sanguin (3) pour qu'ils communiquent entre eux, d'une pompe à liquide de réapprovisionnement (61) qui est agencée dans le passage de liquide de réapprovisionnement (5) et envoie le dialysat du passage d'alimentation en dialysat (23) au circuit sanguin (3), d'un filtre à dialysat (F1) qui est prévu dans le passage d'alimentation en dialysat (23) et purifie le dialysat, d'une pompe à sang (14) qui est agencée dans le passage côté artère (11), et de moyens de commande (1a) qui commandent le fonctionnement de la pompe à liquide de réapprovisionnement (61) et de la pompe à sang (14) ; et
les moyens de commande (1a) sont configurés, lorsque le sang dans le circuit sanguin (3) est retransfusé dans le patient, pour actionner la pompe à liquide de réapprovisionnement (61) pour faire passer un dialysat frais, qui a été purifié par le filtre à dialysat (F1), vers le circuit sanguin (3) à travers le passage de liquide de réapprovisionnement (23) pour retransfuser le sang dans le patient par le biais du dialysat, dans lequel
l'appareil d'hémodialyse comprend en outre des moyens d'ouverture (43) qui ouvrent le circuit de dialysat étanche (4) vers l'extérieur, un récipient de stockage (48) qui stocke à l'intérieur le dialysat frais traversant le passage d'alimentation en dialysat (23), et une source d'énergie auxiliaire (6) pour actionner au moins la pompe à liquide de réapprovisionnement (61), la pompe à sang (14), les moyens d'ouverture (43) et les moyens de commande (1a) ;
**caractérisé en ce que**
un mode de retransfusion de source d'énergie auxiliaire (6) est défini dans les moyens de commande (1a) de sorte à actionner la pompe à liquide de réapprovisionnement (61), la pompe à sang (44) et les moyens d'ouverture (43) par le biais de la source d'énergie auxiliaire (6), à envoyer le dialysat frais stocké dans le récipient de stockage (48) au circuit sanguin (3) à travers le passage de liquide de réapprovisionnement (23), et à retransfuser le sang dans le patient.

2. Appareil d'hémodialyse (1) selon la revendication 1, dans lequel
le passage de liquide de réapprovisionnement (23) est branché entre l'extrémité du passage côté artère (11) reliée au patient et la pompe à sang (14) ;
les moyens de commande (1a) sont configurés pour arrêter la pompe à sang (14) lorsque le sang dans le circuit sanguin (3) est retransfusé depuis le passage côté artère (11) dans le patient, et sont configurés pour actionner la pompe à liquide de réapprovisionnement (61) pour faire passer le dialysat vers le passage côté artère (11) dans une direction opposée à la direction du sang qui a été envoyé ; et
les moyens de commande (1a) sont configurés pour actionner la pompe à liquide de réapprovisionnement (61) et la pompe à sang (14) lorsque le sang est retransfusé dans le patient depuis le passage côté veine (12), et pour faire passer le dialysat du passage côté artère (11) au passage côté veine (12).

3. Appareil d'hémodialyse (1) selon la revendication 2, comprenant en outre des moyens de détection de pression qui sont prévus entre une partie de raccord entre le passage côté artère (11) et le passage de liquide de réapprovisionnement (5), et la pompe à liquide de réapprovisionnement (61).

4. Appareil d'hémodialyse (1) selon la revendication 1, dans lequel
le passage de liquide de réapprovisionnement (5) est branché entre la pompe à sang (14) dans le passage côté artère (11) et le dialyseur (2) ;
les moyens de commande (1a) sont configurés pour actionner la pompe à liquide de réapprovisionnement (61) lorsque le sang dans le circuit sanguin (3) est retransfusé depuis le passage côté artère (11) dans le patient, et sont configurés pour actionner la pompe à sang (14) de sorte à envoyer le sang dans une direction opposée à la direction prise lorsque le sang est envoyé, et pour faire passer le dialysat vers le passage côté artère (11) dans une direction opposée à la direction du sang qui a été envoyé ; et
les moyens de commande (1a) sont configurés pour faire fonctionner la pompe à liquide de réapprovisionnement (61) lorsque le sang est retransfusé depuis le passage côté veine (12) dans le patient, et pour faire passer le dialysat du passage côté artère (11) au passage côté veine (11) .

5. Appareil d'hémodialyse (1) selon la revendication 1, dans lequel
le passage de liquide de réapprovisionnement (5) est relié au passage côté veine (12) ;
les moyens de commande (1a) sont configurés pour actionner la pompe à liquide de réapprovisionnement (61) lorsque le sang dans le circuit sanguin (3) est retransfusé depuis le passage côté artère (12) dans le patient, et sont également configurés pour actionner la pompe à sang (14) de sorte à envoyer le sang dans une direction opposée à la direction prise lorsque le sang est envoyé, et pour faire passer le dialysat du passage côté veine (12) au passage côté artère (11) dans une direction opposée à la direction du sang qui a été envoyé ; et
les moyens de commande (1a) sont configurés pour actionner la pompe à liquide de réapprovisionnement (61) lorsque le sang est retransfusé depuis le passage côté veine (12) dans le patient, et pour faire passer le dialysat vers le passage côté veine (12).
